# EUROPÄISCHE PATENTSCHRIFT

(11) **EP 1 259 218 B1**
(45) Veröffentlichungstag und Bekanntmachung des Hinweises auf die Patenterteilung: **26.05.2010**
(21) Anmeldenummer: 01911453.7
(22) Anmeldetag: 19.02.2001
(51) Int. Cl.: A61Q 19/00, A61K 8/44, A61K 8/55

(54) **KOSMETISCHE ZUSAMMENSETZUNG ZUR BEHANDLUNG VON ALTERNDER UND/ODER GESTRESSTER HAUT**
COSMETIC COMPOSITION FOR THE TREATMENT OF SKIN AGEING AND/OR STRESSED SKIN
COMPOSITION COSMETIQUE POUR LE TRAITEMENT DU VIEILLISSEMENT DE LA PEAU ET/OU DES PEAUX STRESSEES

(30) Priorität: 25.02.2000 DE 10008850
(43) Veröffentlichungstag der Anmeldung: 27.11.2002
(60) Teilanmeldung: 04015242.3
(73) Patentinhaber: Kuhs GmbH, 79541 Lörrach (DE)
(72) Erfinder: ALBRECHT, Martin, 41199 Mönchengladbach (DE)
(74) Vertreter: Lau-Loskill, Philipp
(86) Internationale Anmeldenummer: PCT/DE2001/000615
(87) Internationale Veröffentlichungsnummer: WO 2001/062222

(56) Entgegenhaltungen:
- EP-A- 0 620 000
- WO-A-96/37192
- WO-A-97/00667
- DE-A- 19 839 441
- FR-A- 2 627 385
- FR-A- 2 638 639
- FR-A- 2 713 483
- US-A- 5 610 201
- US-A- 5 834 013
- US-A- 6 015 574

## Beschreibung

Die vorliegende Erfindung betrifft eine kosmetische Zusammensetzung mit den Merkmalen des Oberbegriffs des Patentanspruchs 1.

Kosmetische Zusammensetzungen, insbesondere solche kosmetischen Zusammensetzungen, die zur Pflege und/oder zur Behandlung der Haut und vorzugsweise bei alternder und/oder gestreßter Haut verwendet werden, weisen üblicherweise neben Wasser auch solche Substanzen auf, die mit Wasser Emulsionen ausbilden können. Hierbei stellt eine derartige wäßrige Emulsion, die ggf. noch mit einem lipophilen Zusatz versehen werden kann, den einfachsten Fall einer kosmetischen Zusammensetzung dar.

Darüber hinaus können in einer derartigen bekannten kosmetischen Zusammensetzung noch weitere kosmetische Wirkstoffe, Verdickungsmittel, Gelbildner, Farbstoffe, Stabilisatoren, Alterungsinhibitoren und/oder Duftstoffe sowie pH-Wert-Regulatoren enthalten sein.

Ferner ist es bekannt, daß die im einfachsten Fall einer kosmetischen Zusammensetzung in dieser vorhandenen Substanz unter bestimmten Herstellungsvoraussetzungen mit Wasser lamellare Strukturen ausbilden kann, wobei derartige lamellare Strukturen einen Schichtaufbau besitzen, derart, daß jeweils eine obere Schicht der Substanz zu einer unteren Schicht der Substanz zueinander ausgerichtet ist. Hierbei erfolgt diese Ausrichtung der einzelnen Substanzschichten zueinander abhängig von dem jeweils verwendeten Lösungsmittel derart, daß die hydrophilen Reste der Substanz jeweils nach außen weisen, während die lipophilen Reste zueinander nach innen ausgerichtet sind, oder daß diese lipophilen Reste jeweils nach außen weisen, während die hydrophilen Reste der Substanz nach innen ausgerichtet sind. Letzteres ist immer dann der Fall, wenn das die lamellare Struktur umgebende Medium lipophil ist, während die zuerst beschriebene lamellare Struktur dann vorkommt, wenn die Substanz in einem hydrophilen Medium aufgenommen wird.

Orientieren sich zwei Schichten der Substanz im vorstehenden Sinne, so spricht man von einer Einfachmembran, während bei einer Übereinanderanordnung von zwei Schichtpaaren diese lamellare Struktur dann als Doppelmembran bezeichnet wird.

So beschreibt beispielsweise die US 6,015,574 B eine kosmetische Zusammensetzung insbesondere zur Behandlung von Haaren, die neben einem Phospholipid eine Mischung aus amphoteren und nichtionischen Tensiden enthält.

Aus der US 5,610,201 B ist eine kosmetische oder pharmazeutische Zusammensetzung zur Haar- oder Hautbehandlung bekannt, die neben einem Phospholipid ein kationisches Tensid auf der Basis einer Quärtaren Ammoniumverbindung enthält, wobei die bekannte Zusammensetzung insbesondere als Liposome vorliegt.

Die WO 96/37192 A beschreibt eine kosmetische oder pharmazeutische Zubereitung, die als sehr fein disperse Lösung vorliegt und die neben Sphingolipide oder Glycolipide desweiteren Phospholipide und einen Fettsäureester eines Polyoxyethylen-Sorbitans enthält. Vorzugsweise wird diese bekannte Zubereitung nach Zugabe eines Wirkstoffes auch topisch appliziert.

FR 2 627 385 beschreibt die Verwendung von Phospholipiden, Cholesterin und Methonin zur Herstellung von kosmetischen Zusammensetzungen gegen alternder Haut.

Der vorliegenden Erfindung liegt die Aufgabe zugrunde, eine kosmetische Zusammensetzung zur Verfügung zu stellen, die eine besonders hohe kosmetische Wirksamkeit besitzt.

Diese Aufgabe wird erfindungsgemäß durch eine kosmetische Zusammensetzung mit den kennzeichnenden Merkmalen des Patentanspruchs 1 gelöst.

Die erfindungsgemäße kosmetische Zusammensetzung, die insbesondere bei alternder und/oder gestreßter Haut zur Anwendung gelangt, weist außer Wasser mindestens eine, mit Wasser lamellare Strukturen ausbildende Substanz auf. Desweiteren ist in der erfindungsgemäßen mindestens eine Verbindung, die keine quartäre, synthetische, grenzflächenaktive Ammoniumverbindung ist und die wenigstens eine funktionelle Gruppe der allgemeinen Formel I besitzt

(CH₂ - N^{⊕} - (CH₃)₃ (Formel I)

und/oder S-Adenosylmethionin sowie ein Additiv, ausgewählt aus der Gruppe bestehend aus Methylglycin, Dimethylglycin und Methylmethionin und Mischungen hiervon enthalten.

Mit anderen Worten umfaßt somit die erfindungsgemäßen kosmetische Zusammensetzung im einfachsten Fall neben Wasser, mindestens eine, mit Wasser lamellare Strukturen ausbildende Substanz und die zuvor genannte Verbindung, die die vorstehend aufgeführte funktionelle Gruppe der allgemeinen Formel I besitzt, und/oder S-Adenosylmethionin sowie das zuvor genannte Additiv.

Überraschend konnte festgestellt werden, daß die erfindungsgemäße Zusammensetzung eine hohe kosmetische Wirksamkeit besitzt, die sich einerseits in einer Schutzfunktion der Haut und andererseits in einer Heilfunktion der alternden, erkrankten und/oder gestreßten Haut ausdrückt, so daß die mit der erfindungsgemäßen Zusammensetzung behandelte Haut geschmeidig und glatt ist bzw. wird und bereits gereizte und/oder gestreßte Haut wieder zu ihrem ursprünglichen natürlichen Aussehen und Zustand verhilft. Desweiteren war zu beobachten, daß gealterte Haut in einen gestrafften und elastischen Zustand zurückgeführt wird, so daß insbesondere unerwünschte Falten geglättet werden. Bereits nach wenigen Anwendungen der erfindungsgemäßen kosmetischen Zusammensetzung zeigte trockene Haut oder aufgerauhte Haut wieder ihr natürliches und geschmeidiges Aussehen.

Diese positive und zuvor beispielhaft geschilderte erhöhte kosmetische Wirksamkeit der erfindungsgemäßen kosmetischen Zusammensetzung wird darauf zurückgeführt, daß in der erfindungsgemäßen Zusammensetzung lamellaren Strukturen vorhanden oder während der Anwendung derselben ausgebildet werden, wobei die lamellaren Strukturen insbesondere bei gestörten Barrierefunktionen der Haut in die interzellulären Lipide der Cornea wandern und dort als Kit- und Heilsubstanz wirken. Desweiteren wird angenommen, daß die lamellaren Strukturen das S-Adenosylmethionin und/oder die Verbindung, die wenigstens eine funktionelle Gruppe der allgemeinen Formel I aufweist, und/oder das Additiv an die Störstellen der Hautbarriere herantransportieren, wobei das S-Adenosylrnethonin und/oder die zuvor genannte Verbindung und/oder das Additiv hervorragend geeignet ist bzw. sind, die bei einer Störung der Energieumwandlung in der Zelle auftretenden und zuviel erzeugten Elektronen und/oder die an der falsche Stelle erzeugten Elektronen und/oder die fehlende und/oder zu geringe Sauerstoffzufuhr, die letztendlich auslösende Elemente für alle zuvor aufgeführten Hautbeeinträchtigungen darstellen, abzupuffern. Diese Elektronen und/oder eine gestörte Sauerstoffzufuhr und -versorgung der Zellen der Haut sind aufgrund der Erkenntnisse der vorliegenden Erfindung letztendlich für eine Schädigung der Zellen und damit auch für eine unerwünschte Hautveränderung und/oder Hautschädigung verantwortlich und stellen eine wesentliche Ursache für ein schnelles Altern der Haut dar. Von daher wird auch verständlich, daß die erfindungsgemäße kosmetische Zusammensetzung wirksam das Altern der Haut und die damit verbundenen unerwünschten Hautveränderungen, so beispielsweise Hauterschlaffung oder die Ausgestaltung von Falten, erheblich verzögert. Insbesondere die in der erfindungsgemäßen Zusammensetzung enthaltene Kombination der die lamellaren Strukturen ausbildenden Substanz mit der Verbindung, die wenigstens eine funktionelle Gruppe der allgemeinen Formel I aufweist, mit dem Additiv und/oder mit dem S-Adenosylmethionin stellt sicher, daß das S-Adenosylmethionin und/oder die Verbindung auch tatsächlich dort hingelangt, wo die zuvor beschriebene Abpufferung zu erfolgen hat. Dies wird darauf zurückgeführt, daß das S-Adenosylmethionin und/oder die Verbindung in der die lamellare Strukturen ausbildenden Substanz integriert wird, wobei hier sowohl eine Einlagerung und/oder eine Anlagerung des S-Adenosylmethionins und/oder der Verbindung und/oder des Additivs an der die lamellaren Strukturen bildenden Substanz als auch eine Integration des S-Adeposylmethionins und/oder der Verbindung und/oder des Additivs in die von der Substanz ausgebildeten lamellaren Strukturen im Sinne von misch-lamellaren Strukturen möglich sind.

Desweiteren fungieren das in der erfindungsgemäßen Zusammensetzung vorhandene S-Adenosylmethionin und/oder die wenigstens eine funktionelle Gruppe der allgemeinen Formel I aufweisende Verbindung und/oder das Additiv mittels der darin enthaltenen mindestens einen Methylgruppe als Elektronenakzeptor und als Sauerstoffersatz und fängt somit überschüssige, für den Stoffwechsel der einzelenen Zelle nicht benötigte Elektronen ab, wobei diese Methylgruppe bzw. die entsprechenden Methylgruppen in unschädliches Methan umgewandelt wird bzw. werden.

Diese zuvor bei der erfindungsgemäßen Zusammensetzung beschriebene Wirkung ist für den Durchschnittsfachmann um so erstaunlicher, da S-Adenosylmethionin und auch Verbindungen mit der funktionellen Gruppe gemäß Formel I und auch das Additiv an sich sehr stabil sind und nur unter sehr extremen thermischen Bedingungen außerhalb des Organismusses zerfallen. Hier wird angenommen, daß aufgrund der gezielten und lokalen Anwendung an der jeweils geschädigten Zelle oder den jeweils geschädigten Zellen solche Reaktionsbedingungen geschaffen werden, die die zuvor angesprochene enzymatisch herbeigeführte Umwandlung in Methan ermöglichen.

Zusammenfassend ist somit festzuhalten, daß die erfindungsgemäße kosmetische Zusammensetzung in wirkungsvoller Weise verhindert, daß überschüssige energiereiche Elektronen und deren Folgeprodukte, d.h. insbesondere Sauerstoffradikale überhaupt gebildet werden und/oder daß andere Radikale in unerwünschter Weise auf die Haut, die Haare oder die Nägel eingreifen und hier eine zelluläre Veränderung, Schädigung und/oder Zerstörung bewirken. Von daher greift die erfindungsgemäße Zusammensetzung im biologischen Zyklus der Haut, Haare oder Nägel in eine Vorstufe, d.h. in einer solchen Stufe, in denen Elektronen und/oder von außen einwirkende Radikale im Überschuß vorhanden oder diese Elektronen bzw. Radikale fehlgeleitet sind, ein, so daß dementsprechend diese eliminiert werden. Bedingt dadurch, daß die erfindungsgemäße kosmetische Zusammensetzung dann nach Abfangen der überschüssigen energiereichen Elektronen und/oder Radikale diese in nicht toxische Produkte und Methan umwandelt, wird in besonders wirksamer Weise bei Anwendung der erfindungsgemäßen Zusammensetzung eine Schädigung der Zellen bzw. der Hautbarriere verhindert.

Grundsätzlich ist festzuhalten, daß die erfindungsgemäße kosmetische Zusammensetzung als mit Wasser lamellare Strukturen ausbildende Substanz solche Substanzen enthält, die einen hydrophilen sowie gleichzeitig einen hydrophoben Molekülrest aufweisen. Hier sind insbesondere bevorzugt als Substanz Monoglyceride, Diglyceride, insbesondre destillierte mittelkettige Monoglyceride, Sphingolipide, Phospholipide, Fettalkohole, Fettsäuren, Seifen, Mono- und/oder Di-ester von Fettsäuren, Succrose, Glucose und/oder deren Derivaten, glucosidische, furanosidische und/oder pyranosidische Kondensationsprodukte von Fettalkoholen mit Glucose und/oder Succrose und deren Polymerderivaten, Mono- und/oder Di-ester von Glucosiden mit Fettsäurederivaten, Sterole, Mono- und/oder Di-ester von Fettsäuren und Sterolen und/oder Glycol-Derivate aus Sterolen aufzuführen, wobei die Fettsäuren bevorzugt eine C₈-C₂₂ gesättigte, lineare Kohlenstoffkette besitzen.

Besonders geeignet ist es jedoch, wenn in der erfindungsgemäßen kosmetischen Zusammensetzung als Substanz, die in der Lage ist, mit Wasser lamellare Strukturen auszubilden, mindestens ein hydriertes Phospholipid, und insbesondere ein hydriertes Phosphatidylcholin, enthalten ist. Hier konnte nämlich festgestellt werden, daß derartige hydrierte Phospholipide und insbesondere das hydrierte Phosphatidylcholin einerseits im hohen Maße mit Wasser lamellare Strukturen ausbildet und andererseits diese lamellaren Strukturen hervorragend geeignet sind, in die interzellularen Lipide der Cornea zu wandern und damit gleichzeitig dort große Mengen der in der erfindungsgemäßen kosmetischen Zusammensetzung enthaltenen Verbindung, des Additivs und/oder des S-Adenosylmethionins zur Verfügung zu stellen, wodurch die besonders hohe Wirksamkeit dieser bevorzugten Ausführungsform der erfindungsgemäßen kosmetischen Zusammensetzung erklärlich wird.

Die vorstehend beschriebenen Vorteile besitzen solche Weiterbildungen der erfindungsgemäßen Zusammensetzung im verstärkten Maß, die als Substanz ein hydriertes Phospholipid enthalten, das mindestens 60 Gew.% und vorzugsweise zwischen 70 Gew.% und 95 Gew.% hydriertes Phosphatidylcholin aufweisen, wobei sich diese Konzentrationsangaben auf die Konzentration des hydrierten Phospholipids in der anwendungsfertigen Zusammensetzung beziehen.

Bezüglich der Konzentration der in der erfindungsgemäßen kosmetischen Zusammensetzung enthaltenen mindestens einen Substanz, die mit Wasser lamellare Strukturen ausbilden kann, ist allgemein festzuhalten, daß sich diese Konzentration nach dem Speicher- und Transportvermögen der entsprechend auszubildenden lamellaren Struktur für die Verbindung, das Additiv und/oder für das S-Adenosylmethionin richtet. Insbesondere ist diese mindestens eine Substanz in der erfindungsgemäßen kosmetischen Zusammensetzung in einer Konzentration zwischen 0,01 Gew.% und 10 Gew.%, vorzugsweise in einer Konzentration zwischen 2 Gew.% und 7 Gew.%, vorhanden, wobei sich diese Konzentrationsangaben auf die anwendungsfertige kosmetische Zusammensetzung beziehen.

Bezüglich der in der erfindungsgemäßen. Zusammensetzung enthaltenen Verbindung und/oder des Additivs ist festzuhalten, daß vorzugsweise das Additiv bzw. die Verbindung, die mindestens eine funktionelle Gruppe der vorstehend wiedergegebenen allgemeinen Formel I enthält, ein solches Additiv bzw. eine solche Verbindung sind, die natürlich in aeroben Zellen, insbesondere dort in den Zellmembranen, vorhanden ist. Ausdrücklich soll jedoch an dieser Stelle nochmals betont werden, daß die erfindungsgemäße Zusammensetzung als Verbindung nicht solche chemische Komponenten aufweisen soll, die in der Technik im großen Umfang als quartäre Ammoniumverbindungen bezeichnet werden und die synthetische grenzflächenaktive Substanzen darstellen.

Besonders vorteilhaft ist es, wenn die erfindungsgemäße Zusammensetzung als Verbindung Betain, Acetylcholin, N-Acetyl-Ethanolamin, Cholin, Glycerophosphocholin, Phosphatiylcholin, Lysophosphatidylcholin, Carnitin, Acylcarnitin, Sphingomyeline jeweils allein oder in Mischung untereinander und/oder Derivate hiervon enthalten.

Abhängig vom jeweiligen Anwendungszweck richtet sich bei der erfindungsgemäßen Zusammensetzung die Konzentration der in der erfindungsgemäßen Zusammensetzung enthaltenen Verbindung mit der in Formel I wiedergegebenen funktionellen Gruppe und/oder des Additivs und/oder die Konzentration des S-Adenosylmethionins. Besonders bevorzugt ist es, wenn die Verbindung und/oder das Additiv und/oder das S-Adenosylmethionin in einer Konzentration zwischen 0,0001 % und 10 %, vorzugsweise zwischen 0,1 % und 9 %, jeweils bezogen auf das Gewicht der anwendungsfertigen Zusammensetzung, vorhanden ist.

Eine mit einer besonders hohen kosmetischen Wirksamkeit versehene Ausgestaltung der erfindungsgemäßen kosmetischen Zusammensetzung sieht vor, daß hierbei die Zusammensetzung eine Mischung enthält, die als Inhaltsstoffe Betain, Methylglycin, N-Acetyl-Ethanolamin und/oder Inositol umfaßt. Hierbei kann diese Ausgestaltung wahlweise entweder alle zuvor genannten vier bevorzugten Inhaltsstoffe aufweisen oder nur drei oder zwei Inhaltsstoffe der zuvor genannten Art enthalten, so insbesondere die Kombination von Betain und Methylglycin, die Kombination von N-Acetyl-Ethanolamin und Methylglycin, die Kombination von Inositol und Methylglycin, die Kombination von Betain und N-Acetyl-Ethanolamin sowie die Kombination von Betain mit N-Acetyl-Ethanolamin und Methylglycin.

Weist die zuvor beschriebene Ausgestaltung der erfindungsgemäßen kosmetischen Zusammensetzung eine Mischung aus zwei der zuvor genannten speziellen Inhaltsstoffe auf, so variiert das Mol-Massen-Verhältnis dieser beiden Inhaltsstoffe (Betain, Methylglycin, N-Acetyl-Ethanolamin, Inositol) insbesondere zwischen 1:1 bis 1:9.

Eine besonders geeignete und vorteilhafte Weiterbildung der erfindungsgemäßen kosmetischen Zusammensetzung sieht vor, daß hierbei die erfindungsgemäße kosmetische Zusammensetzung neben Wasser, der die lamellaren Strukturen ausbildenden Substanz, der Verbindung, dem Additiv und/oder dem S-Adenosylmethionin desweiteren mindestens einen kosmetischen Wirkstoff aufweist, wobei insbesondere ein solcher kosmetischer Wirkstoff in der erfindungsgemäßen Zusammensetzung enthalten ist, der die Haut, die Haare und/oder die Nägel pflegt.

Unter den Begriff kosmetischer Wirkstoff fallen insbesondere solche Wirkstoffe, die eine Reinigung und Pflege der Haut und eine Erhaltung des gesunden Hautzustandes, einen äußeren Schutz der Haut vor schädigenden Umwelteinflüssen, klimatischen und aktinischen Einflüssen, so insbesondere bei überschüssiger Sonnen- und UV-Einstrahlung, einen Schutz der Haut vor Wasch- und Reinigungsmittel sowie sonstiger Umweltbelastung, so insbesondere Staub und Emissionen, bewirken. Hierzu gehören insbesondere ungesättigte Fettstoffe, die eine geschmeidigmachende Wirkung besitzen, flüssige Fettsäureester und Kohlenwasserstoffe mit kurzkettiger Verzweigung, die eine spreitenden Wirkung haben, abdeckende und schützende Fettstoffe, die insbesondere Öle, flüssige Fettalkohole, Silikonöle, feste Fettsäureester und/oder Fettalkohole umfassen, wobei vorzugsweise als kosmetischer Wirkstoff in der erfindungsgemäßen kosmetischen Zusammensetzung ein Öl und/oder ein Ölbestandteil, insbesondere auch die unverseifbaren Anteile eines pflanzlichen Öls, wie vorzugsweise Avocadoöl, Olivenöl und/oder mindestens ein natives Öl enthalten ist. Darüber hinaus sind als kosmetische Wirkstoffe auch Vitamine, Olegoproteine, Collagen-Hydrolysate sowie die an sich bekannten und gebräuchlichen UV-Filtersubstanzen zu nennen.

Wird die erfindungsgemäße Zusammensetzung im Bereich der kosmetischen Behandlung von Haaren und Nägel verwendet, so enthalten diese Ausführungsformen der erfindungsgemäßen kosmetischen Zusammensetzung vorzugsweise neben Verdickungs- und Bindemitteln, insbesondere auf der Basis von natürlichen Polymeren, auch rückfettende Substanzen, wie beispielsweise Vaseline, Paraffinöle, Cetylalkohole, Polysiloxane und/oder Lanolin.

Insbesondere variiert die Konzentration der zuvor aufgeführten und in der erfindungsgemäßen Zusammensetzung enthaltenen pflegenden Wirkstoffe zwischen 1 % und 55 % und insbesondere zwischen 5 % und 30 %, bezogen auf das Gewicht der anwendungsfertigen kosmetischen Zusammensetzung.

Bei einer anderen Ausführungsform der erfindungsgemäßen kosmetischen Zusammensetzung weist dieser zusätzlich zu den zuvor aufgeführten pflegenden Wirkstoffe oder anstelle der zuvor aufgeführten pflegenden Wirkstoffe insbesondere noch mindestens einen weiteren Wirkstoff auf, wobei es sich hierbei vorzugsweise um solch einen Wirkstoff handelt, der bei einer topischen Applikation die Feuchtigkeit der Haut erhöht. Als bevorzugtes Beispiel eines derartigen, die Hautfeuchtigkeit erhöhenden Wirkstoffes sind allgemein die N-Acyl-Alkanolamine, so vorzugsweise Lactamid MEA, Oleamid MEA und/oder Acetamid MEA, und insbesondere die N-Acyl-Ethanolamine, so insbesondere N-Acetyl-Phosphatidylethanolamin, das bereits vorstehend mehrfach erwähnte N-Acetyl-Ethanolamin, N-Oleoyl-Ethanolamin, N-Linolenoyl-Ethanolamin sowie N-Acyl-Ethanolamin und/oder N-Acyl-2-Hydroxy-Propylamin, wobei die zuletzt genannten beiden Verbindungen dann als Acylreste Fettsäuren aus Kokosfett und/oder Palmöl enthalten.

Die Konzentration dieser, die Hautfeuchtigkeit erhöhenden Wirkstoffe in der erfindungsgemäßen Zusammensetzung variiert vorzugsweise zwischen 0,5 % und 20 %, jeweils bezogen auf das Gewicht der anwendungsfertigen kosmetischen Zusammensetzung.

Bei einer besonders vorteilhaften Weiterbildung der zuvor beschriebenen Ausführungsformen der erfindungsgemäßen Zusammensetzung weist diese Weiterbildung als Verbindung eine Fettsäure, ein Fettsäuresalz und/oder eine Mischung aus Betain mit mindestens einer Fettsäure und/oder eine Mischung als Betain mit mindestens einem Fettsäuresalz auf.

Vorzugsweise wird hierbei als Fettsäuresalz ein solches Salz ausgewählt, bei dem die zugrundeliegende Fettsäure eine lineare Fettsäure ist und zwischen 12 bis 22 Kohlenstoffatomen aufweist, während bei Verwendung einer Fettsäure diese vorzugsweise ebenfalls zwischen 12 und 22 Kohlenstoffatomen enthält.

Besonderes geeignete Fettsäuresalze des Betains stellen Betainlaurat, Betainmyristat, Betainpalmitat, Betainstearat, Betainoleat und Betainlinolat, jeweils allein oder in Mischung, dar. Hier konnte überraschend festgestellt werden, daß insbesondere diese zuvor konkret genannten Fettsäuresalze des Betains trotz ihrer relativ schlechten Wasserlöslichkeit eine besonders hohe kosmetische Wirksamkeit der erfindungsgemäßen Zusammensetzung verleiht.

Wie bereits eingangs ausgeführt ist, weist die erfindungsgemäßen kosmetische Zusammensetzung Wasser auf, wobei die Konzentration des Wassers in der erfindungsgemäßen Zusammensetzung insbesondere zwischen 5 % und 90 %, bezogen auf das Gewicht der anwendungsfertigen kosmetischen Zusammensetzung, variiert.

Der in der vorliegenden Beschreibung verwendete Begriff Wasser deckt alle wäßrigen Systeme, so insbesondere sterilisiertes Wasser, entionisiertes Wasser, destilliertes Wasser sowie wäßrige Lösungen und/oder wäßrige Puffersysteme, ab.

Desweiteren können, je nach der Art der jeweils gewählten Formulierung, in der erfindungsgemäßen kosmetischen Zusammensetzung mindestens ein Konservierungsmittel, ein Antioxidanz, ein Verdickungsmittel, ein Gelbildner und/oder mindestens ein Alkohol, vorzugsweise ein mehrwertiger Alkohol, enthalten sein.

Eine besonders bevorzugte und vielfältig anzuwendende Ausgestaltung der erfindungsgemäßen Zusammensetzung enthält zwischen
5 % und 90 % Wasser,
0,01 % und 10 % lamellare Strukturen ausbildende Substanz,
0,0001 % bis 10 % der Verbindung und/oder des S-Adenosylmethionins,
0,0001 % bis 10 % des Additivs,
0,5 % bis 20 % die Hautfeuchtigkeit erhöhender Wirkstoff,
1 % bis 55 % des mindestens einen pflegenden Wirkstoffes
sowie übliche sonstige Bestandteile in einer Konzentration zwischen 0 % und 10 %, wobei der zuvor verwendete Begriff sonstige Bestandteile insbesondere Konservierungsmittel, Antioxidantien, Verdickungsmittel, Gelbildner und/oder einen Alkohol, vorzugsweise einen mehrwertigen Alkohol, abdecken. Die zuvor wiedergegebenen Konzentrationsangaben beziehen sich jeweils auf das Gewicht der anwendungsfertigen Zusammensetzung.

Die erfindungsgemäße kosmetische Zusammensetzung kann grundsätzlich in jeder, zur topischen Anwendung geeigneten Formulierung aufgemacht sein, wobei insbesondere die erfindungsgemäßen kosmetische Zusammensetzung als topisch applizierbare Creme formuliert ist. Hierbei weist diese topisch applizierbare Creme eine Viskosität bei 20 °C zwischen 4.000 mPas und 40.000 mPas, vorzugsweise zwischen 12.000 mPas und 25.000 mPas, auf, so daß sich eine derartig formulierte Creme einwandfrei und besonders glatt auf der Haut verteilen läßt.

Um die kosmetische Wirksamkeit der erfindungsgemäßen Zusammensetzung insbesondere auch bei empfindlicher Haut oder bei empfindlichen Haaren bzw. empfindlichen Nägeln sicherzustellen, weist vorzugsweise die erfindungsgemäße Zusammensetzung einen solchen pH-Wert auf, der zwischen 4,0 und 7,2 variiert.

Wie bereits eingangs bei der erfindungsgemäßen Zusammensetzung ausführlich erläutert ist, wird als Ursache für die verbesserte kosmetische Wirksamkeit der erfindungsgemäßen Zusammensetzung angenommen, daß in der erfindungsgemäßen Zusammensetzung mindestens eine solche Substanz enthalten ist, die lamellare Strukturen ausbildet. Insbesondere dann, wenn die Zusammensetzung zwischen 15 Gew. % und 95 Gew.%, vorzugsweise zwischen 30 Gew.% und 95 Gew.%, derartiger lamellarer Strukturen aufweist, wobei sich die zuvor angegebenen Konzentrationen auf das Gewicht der in der erfindungsgemäßen Zusammensetzung enthaltenen Substanz, die in der Lage ist, lamellare Strukturen auszubilden, bezieht, weist eine derartige Ausgestaltung eine besonders hohe kosmetische Wirksamkeit auf, da aufgrund der hohen Konzentration an lamellaren Strukturen die hierin integrierte Verbindung, die wenigstens eine funktionelle Gruppe der allgemeinen Formel I enthält, und/oder das Additiv und/oder das hierin integrierte S-Adenosylmethionin in besonders hohen Konzentrationen und besonders schnell in die interzellularen Lipide der Cornea transportiert werden, so daß dort die eingangs bei der erfindungsgemäßen Zusammensetzung beschriebenen Reaktionen besonders schnell stattfinden können.

Vorzugsweise weist die erfindungsgemäße Zusammensetzung solche lamellaren Strukturen auf, deren Dicke zwischen 20 nm und 3 µm, insbesondere zwischen 40 nm und 1 µm, variiert.

Inositol, wie es vorstehend und nachfolgend genannt ist, ist chemisch korrekt als Inosite (Cyclohexan-1,2,3,4,5,6-hexaole) zu bezeichnen.

Insbesondere wird als Methylmethionin in der erfindungsgemäßen Zusammensetzung sowie bei den nachfolgend beschriebenen Ausführungsbeispielen ein Salz von Methylmethionin und vorzugsweise S-Methyl-DL-Methionin-Sulfoniumchlorid verwendet.

Vorteilhafte weiterbildungen der erfindungsgemäßen Zusammensetzung sind in den Unteransprüchen angegeben.

Die erfindungsgemäße Zusammensetzung wird nachfolgend anhand von Ausführungsbeispielen näher erläutert, wobei die Ausführungsbeispiele 6 bis 9 die vorliegende Erfindung widerspiegeln.

### Ausführungsbeispiel 1

Das Ausführungsbeispiel 1 beschreibt eine Creme für die extrem beanspruchte Altershaut.

Es wird eine kosmetische Zusammensetzung aus den nachfolgend aufgelisteten Inhaltsstoffen erstellt:

| Phase 1 | |
|---|---|
| hydriertes Phosphatidylcholin, Konzentration an hydriertem | |
| Phosphatidylcholin 90 Gew.% | 2,0 g |
| Monoglyceride C 12 | 1,5 g |
| Olivenöl | 17,0 g |
| Cholesterol | 2,0 g |
| Ceramid 3 | 0,1 g |
| Avocadin | 1,0 g |
| Squalen | 1,0 g |
| Pentylenglykol | 5,0 g |
| Palmitinsäure | 1,0 g |
| | |

| Phase 2 | |
|---|---|
| Acetamid MEA | 0,5 g |
| Betain, wasserfrei | 0,8 g |
| Carnitin | 0,5 g |
| Wasser DAB 10 | ad 100,0 g |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 15.000 U/min homogenisiert.

An diese Homogenisierung schloß sich eine fünf Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 8.000 U/min. Unter kontinuierlichem Rühren wurde danach die Mischung auf Raumtemperatur abgekühlt.

### Ausführungsbeispiel 2

Das Ausführungsbeispiel 2 beschreibt eine Bodylotion für die zu Barrierestörungen neigenden Haut, wobei die Bodylotion aus den nachfolgend wiedergegebenen Bestandteilen erstellt wurde:

| Phase 1 | |
|---|---|
| hydriertes Phosphatidylcholin, Konzentration an hydriertem | |
| Phosphatidylcholin 90 Gew.% | 1,5 g |
| Monoglyceride C 10 | 1,2 g |
| Olivenöl | 18,0 g |
| Cholesterol | 1,0 g |
| Ceramid 3 | 0,1 g |
| Squalen | 1,0 g |
| Benzylalkohol | 1,0 g |
| Palmitinsäure | 1,0 g |
| | |

| Phase 2 | |
|---|---|
| Lactamid MEA | 0,5 g |
| Betain, wasserfrei | 1,0 g |
| Cholin, wasserfrei | 0,2 g |
| Wasser DAB 10 | ad 100,0 g |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 10.000 U/min homogenisiert.

An diese Homogenisierung schloß sich eine fünf Minuten dauernde Zwangshomogenisierung mittels Hochdruckhomogenisation bei 600 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 8.000 U/min. Unter kontinuierlichem Rühren wurde danach die Mischung auf Raumtemperatur abgekühlt.

### Ausführungabeiepiel 3

Es wurde ein Bodyspray für die extrem beanspruchte Haut unter Verwendung der nachfolgend aufgeführten Inhaltsstoffe erstellt:

| Phase 1 | |
|---|---|
| hydriertes Phosphatidylcholin, Konzentration an hydriertem | |
| Phosphatidylcholin 90 Gew.% | 2,0 g |
| Monoglyceride C 16 | 1,8 g |
| Olivenöl | 17,0 g |
| Cholesterol | 2,0 g |
| Ceramid 3 | 0,1 g |
| Avocadin | 1,0 g |
| Squalen | 1,0 g |
| Pentylenglykol | 5,0 g |
| Betainpalmitat | 1,8 g |
| | |

| Phase 2 | |
|---|---|
| Acetamid MEA | 0,5 g |
| Wasser DAB 10 | ad 100,0 g |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 15.000 U/min homogenisiert.

An diese Homogenisierung schloß sich eine fünf Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 8.000 U/min. Unter kontinuierlichem Rühren wurde danach die Mischung auf Raumtemperatur abgekühlt.

### Ausführungsbeispiel 4

Das Ausführungsbeispiel 4 beschreibt eine Creme für zu Barrierestörungen neigender Haut.

Es wird eine kosmetische Zusammensetzung aus den nachfolgend aufgelisteten Inhaltsstoffen erstellt:

| Phase 1 | |
|---|---|
| hydriertes Phosphatidylcholin, Konzentration an hydriertem | |
| Phosphatidylchvlin 90 Gew.% | 2,0 g |
| destillierte Monoglyceride C 10 | 1,7 g |
| Olivenöl | 20,0 g |
| Pentylenglykol | 5,0 g |
| Palmitinsäure | 2,2 g |
| | |

| Phase 2 | |
|---|---|
| Palmitamid MEA | 0,5 g |
| Betain, wasserfrei | 0,8 g |
| Cholin | 0,2 g |
| Wasser DAB 10 | ad 100,0 g |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 15.000 U/min homogenisiert.

An diese Homogenisierung schloß sich eine fünf Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 8.000 U/min. Unter kontinuierlichem Rühren wurde danach die Mischung auf Raumtemperatur abgekühlt.

### Ausführungsbeispiele 5 bis 9

Die nachfolgenden Ausführungsbeispiele 5 bis 9 beschreiben eine Creme zur Anwendung sowohl bei gestreßter als auch bei gereizter Haut, insbesondere auch bei gealterter Haut.

Es wurde eine kosmetische Zusammensetzung aus den nachfolgend aufgelisteten Inhaltsstoffen erstellt, wobei alle Ausführungsbeispiele 5 bis 9 identische Phasen 1 und 2, jedoch jeweils eine unterschiedliche Phase 3 aufwiesen.

Die Phase 1 wies folgende, bei den Ausführungsbeispielen 5 bis 9 identische Inhaltsstoffe auf.

| Phase 1 | |
|---|---|
| hydriertes Phosphatidylcholin, Konzentration an hydriertem | |
| Phosphatidylcholin 90 Gew.% | 1,95 % |
| Olivenöl (C 16:0, C 18:1) | 19,2 % |
| C 18:1 Triglyceride | 3,9 % |
| C 16:0 Triglyceride | 3,9 % |
| Squalan | 1,9 % |
| | |

| Phase 2 | |
|---|---|
| Sodium Carbomer | 0,24 % |
| Xanthan Gum | 0,1 % |
| Pentylenglykol | 5,0 % |
| Glycerin | 5,9 % |
| Hydroxyethylcellulose | 0,2 % |
| Wasser | ad 100 % |

Die vorstehend genannten und die nachstehend aufgeführten Prozentangaben beziehen sich alle auf Gew.%.

Das Ausführungsbeispiel 5 beinhaltete eine Phase 3 mit den folgenden Inhaltsstoffen:

| Phase 3 | |
|---|---|
| Acetamid MEA | 0,5 % |
| Betain | 0,3 % |

Das Ausführungsbeispiel 6 beinhaltete eine Phase 3 mit den folgenden Inhaltsstoffen:

| Phase 3 | |
|---|---|
| Acetamid MEA | 0,5 % |
| Methylglycin (Sarkosin) | 0,2 % |

Das Ausführungsbeispiel 7 beinhaltete eine Phase 3 mit den folgenden Inhaltsstoffen:

| Phase 3 | |
|---|---|
| Acetamid MEA | 0,5 % |
| Betain | 0,3 % |
| Methylglycin (Sarkosin) | 0,2 % |

Das Ausführungsbeispiel 8 beinhaltete eine Phase 3 mit den folgenden Inhaltsstoffen:

| Phase 3 | |
|---|---|
| Betain | 0,3 % |
| Methylglycin (Sarkosin) | 0,2 % |

Das Ausführungsbeispiel 9 beinhaltete eine Phase 3 mit den folgenden Inhaltsstoffen:

| Phase 3 | |
|---|---|
| Betain | 0,5 % |
| Methylmethionin | 0,2 % |
| Methylglycin (Sarkosin) | 0,2 % |

Die vorstehend bei den Phasen 3 der Ausführungsbeispiele 5 bis 9 angegebenen Prozentangaben der Inhaltsstoffe beziehen sich auf die anwendungsfertige kosmetische Zusammensetzung.

Zur Herstellung der in den Ausführungsbeispielen 5 bis 9 beschriebenen Zusammensetzungen wurden zunächst die Phasen 1 und 2 jeweils getrennt auf 75 °C erwärmt. Hiernach wurde die Phase 2 zu der Phase 1 unter Beibehaltung der Temperatur langsam zugesetzt, während dabei die Mischung kontinuierlich gerührt wurde. Nachdem eine vollständige Mischung erstellt war, wurde diese Mischung während zwei Minuten unter Verwendung eines Homogenisators (Ultra Turrax) während 16.000 Umdrehungen/min homogenisiert.

Anschließend wurde die aus den Phasen 1 und 2 erstellte Mischung unter kontinuierlichem Rühren auf 37 °C abgekühlt. Hiernach wurde die Phase 3 zu der Mischung der Phasen 1 und 2 unter Beibehaltung der Temperatur langsam zugesetzt, während die Mischung kontinuierlich weiter gerührt wurde.

Hiernach erfolgte eine erneute Homogenisation während fünf Minuten unter Verwendung eines Ultra Turrax-Homogenisators bei 5.000 Umdrehungen/min. Unter kontinuierlichem Rühren wurde hiernach die Mischung auf Raumtemperatur unter Ausbildung der jeweiligen Zusammensetzung abgekühlt.

Die bei den Ausführungsbeispielen verwendete Terminologie der Inhaltsstoffe entspricht so weit es sich hierbei nicht um eindeutige chemische Bezeichnungen handelt der Terminologie, wie sie in "International Cosmetic Ingredient Dictionary and Handbook", 7. Auflage, veröffentlicht von "The Cosmetic, Toiletry, and Fragrance Association, Washington DC" (CTFA), verwendet wird.

## Patentansprüche

1. Kosmetische Zusammensetzung, insbesondere zur Anwendung bei alternder und/oder gestreßter Haut, wobei in der Zusammensetzung außer Wasser mindestens eine, mit Wasser lamellare Strukturen ausbildende Substanz vorhanden ist und die Zusammensetzung desweiteren
a) mindestens eine Verbindung, die keine quartäre, synthetische, grenzflächenaktive Ammoniumverbindung ist und die wenigstens eine funktionelle Gruppe der allgemeinen Formel I enthält
(CH₂ - N^{⊕} - (CH₃)₃ (Formel I),
b) und/oder S-Adenosylmethionin
c) sowie ein Additiv, ausgewählt aus der Gruppe bestehend aus Methylglycin, Dimethylglycin und Methylmethionin und Mischungen hiervon
aufweist.

2. Kosmetische Zusammensetzung nach Anspruch 1, **dadurch gekennzeichnet, daß** die Zusammensetzung als mit Wasser lamellare Strukturen ausbildende Substanz Monoglyceride, Diglyceride, bevorzugt destillierte mittelkettige Monoglyceride, Sphingolipide, Phospholipide, Fettalkohole, Fettsäuren, Seifen, Mono- und/oder Di-ester von Fettsäuren, Succrose, Glucose und/oder deren Derivaten, glucosidische, furanosidische und/oder pyranosidische Kondensationsprodukte von Fettalkoholen mit Glucose und/oder Succrose und deren Polymerderivaten, Mono- und/oder Di-ester von Glucosiden mit Fettsäurederivaten, Sterole, Mono- und/oder Di-ester von Fettsäuren und Sterolen und/oder Glycol-Derivate aus Sterolen enthält.

3. Kosmetische Zusammensetzung nach Anspruch 1 oder 2, **dadurch gekennzeichnet, daß** die Zusammensetzung als mit Wasser lamellare Strukturen ausbildende Substanz ein hydriertes Phospholipid, insbesondere ein hydriertes Phosphatidylcholin, enthält.

4. Kosmetische Zusammensetzung nach Anspruch 3, **dadurch gekennzeichnet, daß** das hydrierte Phospholipid wenigstens 60 Gew.% hydriertes Phosphatidylcholin aufweist.

5. Kosmetische Zusammensetzung nach einem der vorangehenden Ansprüche, **dadurch gekennzeichnet, daß** die Zusammensetzung die mit Wasser lamellare Strukturen ausbildende Substanz in einer Konzentration zwischen 0,01 Gew.% und 10 Gew.%, vorzugsweise in einer Konzentration zwischen 2 Gew.% und 7 Gew.%, bezogen auf die anwendungsfertige Zusammensetzung, aufweist.

6. Kosmetische Zusammensetzung nach einem der vorangehenden Ansprüche, **dadurch gekennzeichnet, daß** die Verbindung Betain, Acetylcholin, N-Acetyl-Ethanolamin, Cholin, Glycerophosphocholin, Phosphatidylcholin, Lysophosphatidylcholin, Carnitin, Acylcarnitin, Sphingomyeline, Mischungen und/oder Derivate davon ist bzw. sind.

7. Kosmetische Zusammensetzung nach einem der vorangehenden Ansprüche, **dadurch gekennzeichnet, daß** die Verbindung und/oder das Additiv und/oder das S-Adenosylmethionin in der Zusammensetzung in einer Konzentration zwischen 0,0001 % und 10 %, vorzugsweise zwischen 0,1 % und 9 %, bezogen auf das Gewicht der anwendungsfertigen Zusammensetzung, vorhanden ist.

8. Kosmetische Zusammensetzung nach einem der vorangehenden Ansprüche, **dadurch gekennzeichnet, daß** die Zusammensetzung eine Mischung enthält, die als Inhaltsstoffe Betain, Methylglycin, N-Acetyl-Ethanolamin und/oder Inositol umfaßt.

9. Kosmetische Zusammensetzung nach Anspruch 8, **dadurch gekennzeichnet, daß** die Mol-Massen in der zwei Inhaltsstoffe aufweisende Mischung von 1:1 bis 1:9 variiert.

10. Kosmetische Zusammensetzung nach einem der vorangehenden Ansprüche, **dadurch gekennzeichnet, daß** die kosmetische Zusammensetzung desweiteren noch mindestens einen kosmetischen Wirkstoff enthält.

11. Kosmetische Zusammensetzung nach Anspruch 10, **dadurch gekennzeichnet, daß** der Wirkstoff ein die Haut, die Haare und/oder die Nägel pflegender Wirkstoff ist.

12. Kosmetische Zusammensetzung nach Anspruch 10 oder 11, **dadurch gekennzeichnet, daß** der Wirkstoff ein Öl und/oder ein Ölbestandteil, insbesondere die unverseifbaren Anteile des Avocadoöls, Olivenöl und/oder mindestens ein natives Öl, ist bzw. sind.

13. Kosmetische Zusammensetzung nach einem der Ansprüche 10 bis 12, **dadurch gekennzeichnet, daß** der pflegende Wirkstoff in einer Konzentration zwischen 1 % und 55 %, bezogen auf das Gewicht der anwendungsfertigen kosmetischen Zusammensetzung, vorhanden ist.

14. Kosmetische Zusammensetzung nach einem der Ansprüche 10 bis 13, **dadurch gekennzeichnet, daß** der Wirkstoff ein die Hautfeuchtigkeit erhöhender Wirkstoff ist.

15. Kosmetische Zusammensetzung nach Anspruch 14, **dadurch gekennzeichnet, daß** der Wirkstoff ein Amid, insbesondere ein Alkanolamid und vorzugsweise Lactamid MEA, Oleamid MEA und/oder ein Acetamid MEA, ist.

16. Kosmetische Zusammensetzung nach Anspruch 14 oder 15, **dadurch gekennzeichnet, daß** der die Hautfeuchtigkeit erhöhende Wirkstoff in der Zusammensetzung in einer Konzentration zwischen 0,5 % .und 20 %, bezogen auf das Gewicht der anwendungsfertigen kosmetischen Zusammensetzung, vorhanden ist.

17. Kosmetische Zusammensetzung nach einem der vorangehenden Ansprüche, **dadurch gekennzeichnet, daß** die kosmetische Zusammensetzung als Verbindung eine Fettsäure, ein Fettsäuresalz und/oder eine Mischung aus Betain mit mindestens einer Fettsäure und/oder einem Fettsäuresalz enthält.

18. Kosmetische Zusammensetzung nach einem der vorangehenden Ansprüche, **dadurch gekennzeichnet, daß** die kosmetische Zusammensetzung als Verbindung mindestens ein Fettsäuresalz des Betains aufweist.

19. Kosmetische Zusammensetzung nach Anspruch 17 oder 18, **dadurch gekennzeichnet, daß** die Fettsäure bzw. das Fettsäuresalz eine Kohlenstoffhauptkette mit 12 bis 22 Kohlenstoffatomen besitzt.

20. Kosmetische Zusammensetzung nach Anspruch 18 oder 19, **dadurch gekennzeichnet, daß** in der kosmetischen Zusammensetzung als Fettsäuresalz des Betains eine Betainlaurat, ein Betainmyristat, ein Betainpalmitat, ein Betainstearat, ein Betainoleat und/oder ein Betainlinolat vorhanden ist.

21. Kosmetische Zusammensetzung nach einem der vorangehenden Ansprüche, **dadurch gekennzeichnet, daß** die Zusammensetzung Wasser in einer Konzentration zwischen 5 % und 90 %, bezogen auf das Gewicht der anwendungsfertigen kosmetischen Zusammensetzung aufweist.

22. Kosmetische Zusammensetzung nach einem der vorangehenden Ansprüche, **dadurch gekennzeichnet, daß** die Zusammensetzung desweiteren mindestens ein Konservierungsmittel, ein Antioxidanz, ein Verdickungsmittel, einen Gelbildner und/oder einen Alkohol, vorzugsweise einen mehrwertigen Alkohol, aufweist.

23. Kosmetische Zusammensetzung nach einem der vorangehenden Ansprüche, **dadurch gekennzeichnet, daß** die kosmetische Zusammensetzung zwischen
5 % und 90 % Wasser,
0,01 % und 10 % lamellare Strukturen ausbildende Substanz,
0,0001 % bis 10 % der Verbindung und/oder des S-Adenosylmethionins,
0,0001 % bis 10 % des Additivs,
0,5 % bis 20 % die Hautfeuchtigkeit erhöhender Wirkstoff,
1 % bis 55 % des mindestens einen pflegenden Wirkstoffes
sowie übliche sonstige Bestandteile in einer Konzentration zwischen 0 % und 10 % enthält; wobei sich die zuvor wiedergegebenen Konzentrationsangaben auf das Gewicht der anwendungsfertigen Zusammensetzung beziehen.

24. Kosmetische Zusammensetzung nach einem der vorangehenden Ansprüche, **dadurch gekennzeichnet, daß** die Zusammensetzung als topisch applizierbare Creme formuliert ist und eine Viskosität bei 20 °C zwischen 4.000 mPas und 40.000 mPas, vorzugsweise zwischen 12.000 mPas und 25.000 mPas, aufweist.

25. Kosmetische Zusammensetzung nach einem der vorangehenden Ansprüche, **dadurch gekennzeichnet, daß** die Zusammensetzung einen pH-Wert zwischen 4,0 und 7,2 besitzt.

26. Kosmetische Zusammensetzung nach einem der vorangehenden Ansprüche, **dadurch gekennzeichnet, daß** die Zusammensetzung lamellare Strukturen aufweist, wobei die Konzentration dieser lamellaren Struktur zwischen 15 % und 95 %, vorzugsweise zwischen 30 % und 95 %, bezogen auf das Gewicht der in der Zusammensetzung enthaltenen und die lamellaren Strukturen ausbildenden Substanz, aufweist.

27. Kosmetische Zusammensetzung nach einem der vorangehenden Ansprüche, **dadurch gekennzeichnet, daß** die Zusammensetzung solche lamellaren Strukturen aufweist, deren Dicke zwischen 20 nm und 3 µm, vorzugsweise zwischen 40 nm und 1 µm, variiert.

## Claims

1. A cosmetic composition, especially for use on aged and/or stressed skin, whereby apart from water at least one substance forming lamellar structures with water is present in the composition, and the composition further contains
a) at least one compound, excluding synthetic quaternary ammonium surfactants, having at least one functional group of general formula I
(CH₂ - N^{⊕} - (CH₃)₃ (Formula I)
b) and/or S-adenosylmethionine,
c) and/or an additiv selected from the group consisting of methyl glycine, dimethylglycine and methyl methionine and mixtures thereof.

2. The cosmetic composition according to claim 1, **characterized in that** the composition contains monoglyceride, diglyceride, preferably distilled medium-chain monoglyceride, sphingolipids, phospholipids, fatty alcohols, fatty acids, soaps, mono- and/or di-esters of fatty acids, succrose, glucose and/or their derivates, glucosidic, furanosidic and/or pyranosidic condensation products of fatty alcohols with glucose and/or succrose and their polymer derivates, mono- and/or di-esters of glucosides with fatty acids derivates, sterols, mono- and/or di-esters of fatty acids and sterols and/or glycol derivates of sterols as a substance forming lamellar structures with water.

3. The cosmetic composition according to claim 1 or 2, **characterized in that** the composition contains a hydrogenated phospholipid, in particular a hydrogenated phosphatidylcholine as a substance forming lamellar structures with water.

4. The cosmetic composition according to claim 3, **characterized in that** the hydrogenated phospholipid has at least 60 % by weight of hydrogenated phosphatidylcholine.

5. The cosmetic composition according to one of the preceding claims, **characterized in that** the composition has the substance forming lamellar structures with water in a concentration between 0,01 % by weight and 10 % by weight, preferably in a concentration between 2 % by weight and 7 % by weight, relative to the ready-to-use composition.

6. The cosmetic composition according to one of the preceding claims, **characterized in that** the compound is betaine, acetyl-choline, N-acetyl-ethanolamine, choline, glycerophosphocholine, phosphatidylcholine, lysophosphatidylcholine, carnitine, acylcarnitine, sphingomyeline, mixtures and/or derivates thereof.

7. The cosmetic composition according to one of the preceding claims, **characterized in that** the compound and/or the additive and/or the S-adenosylmethionine is present in the composition in a concentration between 0,0001 % and 10 and preferably between 0,1 % and 9 %, relative to the weight of the ready-to-use composition.

8. The cosmetic composition according to one of the preceding claims, **characterized in that** the composition contains a mixture including betaine, methyl glycine, N-acetyl-ethanolamine and/or inositol as constituents.

9. The cosmetic composition according to claim 8, **characterized in that** the mol masses in the mixture having two constituents vary from 1:1 to 1:9.

10. The cosmetic composition according to one of the preceding claims, **characterized in that** the cosmetic composition also contains at least one cosmetic active ingredient.

11. The cosmetic composition according to claim 10, **characterized in that** the active ingredient is such an active ingredient used for skin care, hair care and/or nail care.

12. The cosmetic composition according to claim 10 or 11, **characterized in that** the active ingredient is an oil and/or an oil constituent, in particular the unsaponifiable portions of avocado oil, olive oil, and/or at least one native oil.

13. The cosmetic composition according to claims 10 to 12, **characterized in that** the active ingredient, used for skin care, hair care and/or nail care is present in a concentration between 1 % and 55 %, relative to the weight of the ready-to-use cosmetic composition.

14. The cosmetic composition according to claims 10 to 13, **characterized in that** the active ingredient is an active ingredient increasing skin moisture.

15. The cosmetic composition according to claim 14, **characterized in that** the active ingredient is an amide, in particular an alkanolamide and more preferably lactamide MEA, oleamide MEA and/or an acetamide MEA.

16. The cosmetic composition according to claim 14 or 15, **characterized in that** the active ingredient increasing the skin moisture is present in the composition in a concentration between 0,5 % and 20 %, relative to the weight of the ready-to-use cosmetic composition.

17. The cosmetic composition according to one of the preceding claims, **characterized in that** the cosmetic composition contains a fatty acid, a fatty acid salt and/or a mixture of betaine with at least one fatty acid and/or a fatty acid salt as compound.

18. The cosmetic composition according to one of the preceding claims, **characterized in that** the cosmetic composition has at least one fatty acid salt of betaine as compound.

19. The cosmetic composition according to claim 17 or 18, **characterized in that** the fatty acid or the fatty acid salt has a main carbon chain with 12 to 22 carbon atoms.

20. The cosmetic composition according to claim 18 or 19, **characterized in that** betaine laurate, betaine myristate, betaine palmitate, betaine stearate, betaine oleate and/or betaine linolate is present in the cosmetic composition as a fatty acid salt of betaine.

21. The cosmetic composition according to one of the preceding claims, **characterized in that** the composition contains water in a concentration between 5 % and 90 %, relative to the weight of the ready-to-use cosmetic composition.

22. The cosmetic composition according to one of the preceding claims, **characterized in that** the composition additionally has at least one preservative, an antioxidant, a thickener, a gel-forming agent and/or an alcohol, preferably a multivalent alcohol.

23. The cosmetic composition according to one of the preceding claims, **characterized in that** the cosmetic composition contains between
5 % and 90 % water,
0,01 % and 10 % of the substance forming lamellar structures,
0,0001 % to 10 % of the compound and/or the S-adenosylmethionine,
0,0001 % to 10 % of the additive,
0,5 % to 20 % of the active ingredient increasing skin moisture,
1 % to 55 % of the at least one active ingredient, used for skin care, hair care and/or nail care, as well as the usual other constituents in a concentration between 0 % and 10 %,
whereby the above mentioned concentrations are related to the weight of the ready-to-use composition.

24. The cosmetic composition according to one of the preceding claims, **characterized in that** the composition is formulated as a topically applied cream and has a viscosity at 20 °C between 4000 mPas and 40000 mPas, preferably between 12000 mPas and 25000 mPas.

25. The cosmetic composition according to one of the preceding claims, **characterized in that** the composition has a pH value between 4,0 and 7,2.

26. The cosmetic composition according to one of the preceding claims, **characterized in that** the composition has lamellar structures, whereby the concentration of this lamellar structure is between 15 % and 95 %, preferably between 30 % and 95 %, relative to the weight of the substance contained in the composition and forming the lamellar structures.

27. The cosmetic composition according to one of the preceding claims, **characterized in that** the composition has such lamellar structures whose thickness varies between 20 nm and 3 µm, preferably between 40 nm and 1 µm.

## Revendications

1. Composition cosmétique destinée, en particulier, à une application sur une peau vieillissante et/ou stressée, dans laquelle est présente dans la composition, outre de l'eau, au moins une substance formant des structures lamellaires avec l'eau, la composition comprenant en outre :
a) au moins un composé qui n'est pas un composé d'ammonium quaternaire synthétique tensioactif et qui contient au moins un groupement fonctionnel de formule générale I :
(CH₂ - N^{⊕} - (CH₃)₃ (Formule I),
b) et/ou de la S-adénosylméthionine
c) ainsi qu'un additif choisi dans le groupe constitué de la méthylglycine, de la diméthylglycine et de la méthylméthionine et de leurs mélanges.

2. Composition cosmétique selon la revendication 1, **caractérisée en ce que** la composition contient, comme substance formant des structures lamellaires avec l'eau, des monoglycérides, des diglycérides, de préférence des monoglycérides à chaîne moyenne distillés, des sphingolipides, des phospholipides, des alcools gras, des acides gras, des savons, des mono- et/ou di-esters d'acides gras, du saccharose, du glucose et/ou leurs dérivés, des produits de condensation glucosidiques, furanosidiques et/ou pyranosidiques d'alcools gras avec du glucose et/ou du saccharose et leurs dérivés polymères, des mono- et/ou di-esters de glucosides avec des dérivés d'acides gras, des stérols, des mono- et/ou di-esters d'acides gras et de stérols et/ou de dérivés de glycol issus de stérols.

3. Composition cosmétique selon la revendication 1 ou 2, **caractérisée en ce que** la composition contient, comme substance formant des structures lamellaires avec de l'eau, un phospholipide hydrogéné, en particulier, une phosphatidylcholine hydrogénée.

4. Composition cosmétique selon la revendication 3, **caractérisée en ce que** le phospholipide hydrogéné présente au moins 60 % en poids de phosphatidylcholine hydrogénée.

5. Composition cosmétique selon l'une quelconque des revendications précédentes, **caractérisée en ce que** la composition présente la substance formant des structures lamellaires avec l'eau en concentration comprise entre 0,01 % en poids et 10 % en poids, de préférence, en concentration comprise entre 2 % en poids et 7 % en poids par rapport à la composition prête à l'emploi.

6. Composition cosmétique selon l'une quelconque des revendications précédentes, **caractérisée en ce que** le composé est représenté par la bétaïne, l'acétylcholine, la N-acétyléthanolamine, la choline, la glycérophosphocholine, la phosphatidylcholine, la lysophosphatidylcholine, la carnitine, l'acylcarnitine, des sphingomyélines, ainsi que leurs mélanges et/ou dérivés.

7. Composition cosmétique selon l'une quelconque des revendications précédentes, **caractérisée en ce que** le composé et/ou l'additif et/ou la S-adénosylméthionine sont présents dans la composition en concentration comprise entre 0, 0001 % et 10 %, de préférence, entre 0, 1 % et 9 % par rapport au poids de la composition prête à l'emploi.

8. Composition cosmétique selon l'une quelconque des revendications précédentes, **caractérisée en ce que** la composition contient un mélange qui comprend, comme ingrédients, de la bétaïne, de la méthylglycine, de la N-acétyléthanolamine et/ou de l'inositol.

9. Composition cosmétique selon la revendication 8, **caractérisée en ce que** les masses en mole dans le mélange présentant deux ingrédients varient dans un rapport de 1:1 à 1:9.

10. Composition cosmétique selon l'une quelconque des revendications précédentes, **caractérisée en ce que** la composition cosmétique contient en outre encore au moins un principe actif cosmétique.

11. Composition cosmétique selon la revendication 10, **caractérisée en ce que** le principe actif est un principe actif de soin de la peau, des cheveux et/ou des ongles.

12. Composition cosmétique selon la revendication 10 ou 11, **caractérisée en ce que** le principe actif est représenté par une huile et/ou un constituant d'huile, en particulier les fractions insaponifiables de l'huile d'avocat, l'huile d'olive et/ou au moins une huile native.

13. Composition cosmétique selon l'une quelconque des revendications 10 à 12, **caractérisée en ce que** le principe actif de soin est présent en concentration comprise entre 1 % et 55 % par rapport au poids de la composition cosmétique prête à l'emploi.

14. Composition cosmétique selon l'une quelconque des revendications 10 à 13, **caractérisée en ce que** le principe actif est un principe actif augmentant l'hydratation de la peau.

15. Composition cosmétique selon la revendication 14, **caractérisée en ce que** le principe actif est un amide, en particulier, un alcanolamide et, de préférence, le lactamide MEA, l' oléamide MEA et/ou un acétamide MEA.

16. Composition cosmétique selon la revendication 14 ou 15, **caractérisée en ce que** le principe actif augmentant l'hydratation de la peau est présent dans la composition en concentration comprise entre 0, 5 % et 20 % par rapport au poids de la composition cosmétique prête à l'emploi.

17. Composition cosmétique selon l'une quelconque des revendications précédentes, **caractérisée en ce que** la composition cosmétique contient, comme composé, un acide gras, un sel d'acide gras et/ou un mélange de bétaïne et d'au moins un acide gras et/ou d'un sel d'acide gras.

18. Composition cosmétique selon l'une quelconque des revendications précédentes, **caractérisée en ce que** la composition cosmétique présente, comme composé, au moins un sel d'acide gras de la bétaïne.

19. Composition cosmétique selon la revendication 17 ou 18, **caractérisée en ce que** l'acide gras ou le sel d'acide gras présente une chaîne principale carbonée ayant 12 à 22 atomes de carbone.

20. Composition cosmétique selon la revendication 18 ou 19, **caractérisée en ce que** l'on trouve dans la composition cosmétique, comme sel d'acide gras de la bétaïne, un laurate de bétaïne, un myristate de bétaïne, un palmitate de bétaïne, un stéarate de bétaïne, un oléate de bétaïne et/ou un linoléate de bétaïne.

21. Composition cosmétique selon l'une quelconque des revendications précédentes, **caractérisée en ce que** la composition présente de l'eau en concentration comprise entre 5 % et 90 % par rapport au poids de la composition cosmétique prête à l'emploi.

22. Composition cosmétique selon l'une quelconque des revendications précédentes, **caractérisée en ce que** la composition présente en outre au moins un conservateur, un antioxydant, un agent épaississant, un agent gélifiant et/ou un alcool, de préférence, un alcool polyvalent.

23. Composition cosmétique selon l'une quelconque des revendications précédentes, **caractérisée en ce que** la composition cosmétique contient :
entre 5 % et 90 % d'eau,
entre 0,01 % et 10 % d'une substance formant des structures lamellaires,
0,0001 % à 10 % du composé et/ou de la S-adénosylméthionine,
0,0001 % à 10 % de l'additif,
0,5 % à 20 % du principe actif augmentant l'hydratation de la peau,
1 % à 55 % du au moins un principe actif de soin
ainsi que des constituants courants en concentration comprise entre 0 % et 10 % ; les données de concentration exposées au préalable se référant au poids de la composition prête à l'emploi.

24. Composition cosmétique selon l'une quelconque des revendications précédentes, **caractérisée en ce que** la composition est formulée sous la forme d'une crème applicable par voie topique et présente une viscosité à 20 °C comprise entre 4000 mPas et 40 000 mPas, de préférence, entre 12 000 mPas et 25 000 mPas.

25. Composition cosmétique selon l'une quelconque des revendications précédentes, **caractérisée en ce que** la composition présente une valeur de pH comprise entre 4,0 et 7,2.

26. Composition cosmétique selon l'une quelconque des revendications précédentes, **caractérisée en ce que** la composition présente des structures lamellaires, dans laquelle la concentration de cette structure lamellaire est comprise entre 15 % et 95 %, de préférence, entre 30 % et 95 % par rapport au poids de la substance contenue dans la composition et formant les structures lamellaires.

27. Composition cosmétique selon l'une quelconque des revendications précédentes, **caractérisée en ce que** la composition présente ces structures lamellaires, dont l'épaisseur varie entre 20 nm et 3 µm, de préférence, entre 40 nm et 1 µm.
